# EUROPEAN PATENT APPLICATION

(11) **EP 4 371 550 A1**
(43) Date of publication of application: **22.05.2024**
(21) Application number: 23210497.6
(22) Date of filing: 17.11.2023
(51) Int. Cl.: A61K 8/31, A61K 8/33, A61K 8/34, A61K 8/37, A61K 8/46, A61K 8/92, A61Q 13/00, A61Q 15/00

(54) **FRAGRANCE COMPOSITION**

(30) Priority: 21.11.2022 JP 2022185399
(71) Applicant: Takasago International Corporation, Tokyo 144-8721 (JP)
(72) Inventor: MIHARA, Hisashi, Hiratsuka-shi, Kanagawa 2540073 (JP); KASHIWAGI, Takahiro, Hiratsuka-shi, Kanagawa 2540073 (JP); KUWAHARA, Yukari, Hiratsuka-shi, Kanagawa 2540073 (JP)
(74) Representative: Hoffmann Eitle

(57) **Abstract**

The present invention relates to provide a fragrance composition that reduces sensually sufficient discomfort due to at least one malodor of body odor, scalp odor, bathroom odor, and nursing care odor all of which contains hexanal odor, with a wide range of scent tones, without masking the odor with a strong scent. In the present invention, provided is a fragrance composition containing at least one selected from the group consisting of specific fragrance components, preferably a fragrance composition further containing, in combination, at least one selected from the group consisting of other specific fragrance components.

## Description

### TECHNICAL FIELD

The present invention relates to a fragrance composition containing a fragrance component that prevents malodor. The present invention also relates to a product containing the fragrance composition and a method for preventing malodor.

### BACKGROUND ART

In recent years, as consumer preferences and living environments have diversified, the number of people who are sensitive to smell and malodor around them has increased. Body odor (Patent Literature 1), scalp odor (Patent Literature 2), bathroom odor (Patent Literature 3), nursing care odor (Patent Literature 4), and the like are known as discomfortable odor in living spaces, all of which contain hexanal as a causative substance. Hexanal is a type of chain aliphatic aldehyde, and is known as a substance that causes grassy odor of soybeans and grass, and even in the case of odor in living spaces other than food, the presence of the grassy odor increases discomfort.

As a deodorizing technology for bathroom odor that is malodor containing hexanal as a causative substance, Patent Literature 3 discloses a fragrance composition containing fragrance components selected from spearmint oil, peppermint oil, menthol, menthone, isomenthone, and carvone, and all of these have similar scent tones such as peppermint and herbs, and have strong scents that mask malodor.

In addition, as a selective deodorizing technology for malodor, the possibility of a deodorizing method based on an antagonistic mechanism for searching for olfactory receptors that respond to specific odorants and preventing the activation of the olfactory receptors with specific substances is becoming clear. Non Patent Literature 1 discloses OR2W1 as an olfactory receptor that responds to hexanal, which is a malodor-causative substance as an off-flavor of soybean, and discloses that hexanal odor can be prevented by preventing the response of the olfactory receptor. This related-art method is said to specifically prevent recognition of malodor by inhibiting olfactory receptors that respond to hexanal with specific odor molecules.

However, in the related-art technologies, there have been problems in that the range of product scents is limited, the malodor is masked by the strong scent, and the deodorizing effect is not sensually sufficient.

### CITATION LIST

### PATENT LITERATURE

Patent Literature 1: JP2004-263102A
Patent Literature 2: JP2011-184469A
Patent Literature 3: JP2007-014749A
Patent Literature 4: JP2017-165959A

### NON-PATENT LITERATURE

Non Patent Literature 1: Soybean protein research Vol. 23 (2020)

### SUMMARY OF INVENTION

The resent invention relates to provide a technology capable of reducing discomfort based on deodorization of hexanal odor. As a preferable embodiment, the present invention relates to provide a fragrance composition that reduces sensually sufficient discomfort due to at least one malodor of body odor, scalp odor, bathroom odor, and nursing care odor all of which contains hexanal odor, with a wide range of scent tones, without masking the odor with a strong scent.

After conducting intensive studies to solve the above problems, the present inventor has found that specific compounds and essential oil have an effect of reducing discomfort due to at least one selected from the group consisting of body odor, scalp odor, bathroom odor, and nursing care odor all of which contains hexanal odor. More surprisingly, it has been found that by using a specific compound and essential oil in combination, an even better effect of reducing discomfort due to at least one selected from the group consisting of body odor, scalp odor, bathroom odor, and nursing care odor.

That is, the present invention provides a fragrance composition, a product containing the fragrance composition, a method for preventing malodor, a use of a fragrance composition for reducing discomfort due to malodor as shown below.
<1> A fragrance composition for reducing discomfort due to malodor, the fragrance composition including: at least one selected from the group consisting of fragrance components in the following group A.
   Group A: 3-(menthoxy)propane-1,2-diol (COOLACT10), ethyl 3-mercapto-2-methylbutyrate (Guava coeur), dimethyl-2-(1-phenylethyl)cyclopropylmethanol (Pamplefix), 5-cyclohexadecenone (Ambretone), ethyl 2,2,6-trimethylcyclohexane-1-carboxylate (Thesaron), isopulegol, 2-methyl-4-(2,2,3-trimethyl-3-cyclopentenyl)-2-buten-1-ol (Hindinol), 1-(2,2,6-trimethylcyclohexyl)hexan-3-ol (Dextramber), 2-acetyl-2,3,8,8-tetramethyloctarine (Orbitone), p-mentha-1,5-diene (phellandrene), methyl (2-pentyl-3-oxo-cyclopentyl) acetate (Hedione), 3-methyl-4-(2,6,6-trimethyl-2-cyclohexen-1-yl)-3-buten-2-one (α-isomethylionone), cedar leaf oil, bergamot oil, cardamom oil, davana oil, gaiac wood oil, and vetiver oil
<2> The fragrance composition according to <1>, in which the malodor is at least one selected from the group consisting of body odor, scalp odor, bathroom odor, and nursing care odor.
<3> The fragrance composition according to <1>, in which the malodor is hexanal odor.
<4> The fragrance composition according to any one of <1> to <3>, further including: at least one selected from the group consisting of fragrance components in the following group B.
   Group B: limonene, linalool, linalyl acetate, geraniol, citronellal, β-damascone, γ-decalactone, prenyl acetate, isoamyl acetate, benzyl acetate, 3-methyl-2-(cis-2-pentenyl)-2-cyclopenten-1-one, citronellol, allyl hexanoate, ethyl hexanoate, ethyl isovalerate, cis-2-methyl-4-propyl-1,3-oxathiane, ethyl amyl ketone, para-menth-1,8-dien-7-al, (3-methylbutyloxy)-2-propenyl acetate, cis-3-hexenyl acetate, 2-phenylpropanal, citronella java oil, grapefruit oil, nutmeg oil, coriander oil, and galbanum oil
<5> The fragrance composition according to <4>, in which the fragrance component in the group A is contained in an amount of 0.000001 mass% to 50 mass%, and the fragrance component in the group B is contained in an amount of 0.0001 mass% to 50 mass%.
<6> A product including: the fragrance composition according to any one of <1> to <5> in an amount of 0.01 mass% to 100 mass%.
<7> A method for reducing discomfort due to at least one of malodor selected from the group consisting of body odor, scalp odor, bathroom odor, and nursing care odor, the method including: applying the fragrance composition according to any one of <1> to <5> to at least one of malodor selected from the group consisting of body odor, scalp odor, bathroom odor, and nursing care odor to reduce discomfort due to malodor.
<8> A use of a fragrance composition for reducing discomfort due to malodor, the fragrance composition including: at least one selected from the group consisting of fragrance components in the following group A.
   Group A: 3-(menthoxy)propane-1,2-diol, ethyl 3-mercapto-2-methylbutyrate, dimethyl-2-(1-phenylethyl)cyclopropylmethanol, 5-cyclohexadecenone, ethyl 2,2,6-trimethylcyclohexane-1-carboxylate, isopulegol, 2-methyl-4-(2,2,3-trimethyl-3-cyclopentenyl)-2-buten-1-ol, 1-(2,2,6-trimethylcyclohexyl)hexan-3-ol, 2-acetyl-2,3,8,8-tetramethyloctarine, p-mentha-1,5-diene, methyl (2-pentyl-3-oxo-cyclopentyl) acetate, 3-methyl-4-(2,6,6-trimethyl-2-cyclohexen-1-yl)-3-buten-2-one, cedar leaf oil, bergamot oil, cardamom oil, davana oil, gaiac wood oil, and vetiver oil
<9> The use of the fragrance composition for reducing discomfort due to malodor according to <8>, in which the malodor is at least one selected from the group consisting of body odor, scalp odor, bathroom odor, and nursing care odor.
<10> The use of the fragrance composition for reducing discomfort due to malodor according to <8>, wherein the malodor is hexanal odor.
<11> The use of the fragrance composition for reducing discomfort due to malodor according to any one of <8> to <10>, the fragrance composition further including: at least one selected from the group consisting of fragrance components in the following group B.
   Group B: limonene, linalool, linalyl acetate, geraniol, citronellal, β-damascone, γ-decalactone, prenyl acetate, isoamyl acetate, benzyl acetate, 3-methyl-2-(cis-2-pentenyl)-2-cyclopenten-1-one, citronellol, allyl hexanoate, ethyl hexanoate, ethyl isovalerate, cis-2-methyl-4-propyl-1,3-oxathiane, ethyl amyl ketone, para-menth-1,8-dien-7-al, (3-methylbutyloxy)-2-propenyl acetate, cis-3-hexenyl acetate, 2-phenylpropanal, citronella java oil, grapefruit oil, nutmeg oil, coriander oil, and galbanum oil

According to the present invention, a fragrance composition containing at least one selected from the group consisting of the group A can reduce discomfort due to at least one of body odor, scalp odor, bathroom odor, and nursing care odor all of which contains hexanal odor. Further, by using at least one selected from the group B in combination, it is possible to provide a better effect of reducing discomfort than using only the group A.

### DESCRIPTION OF EMBODIMENTS

A fragrance composition according to the present invention contains at least one from the above group A as an effective component, and more preferably contains at least one from each of the two groups A and B as effective components.

A blending amount of the group A in the fragrance composition according to the present invention is not limited depending on a product or a scent tone, and the blending amount of a compound having a low threshold such as Guava coeur is preferably 0.000001 mass% or more, more preferably in a range of 0.0001 mass % to 1 mass%, and still more preferably 0.0001 mass% to 0.01 mass%. The blending amount of another component such as bergamot oil is preferably 0.1 mass% or more, more preferably in a range of 0.1 mass% to 50 mass%, and still more preferably 0.1 mass% to 20 mass%.

A blending amount of the group B in the fragrance composition according to the present invention is not limited depending on a product or a scent tone, and the blending amount of a compound having a low threshold such as cis-2-methyl-4-propyl-1,3-oxathiane is preferably 0.0001 mass% or more, more preferably in a range of 0.001 mass% to 1 mass%, and still more preferably 0.001 mass% to 0.01 mass%. The blending amount of another component such as grapefruit oil is preferably 0.1 mass %or more, more preferably in a range of 0.1 mass% to 50 mass%, and still more preferably 0.1 mass% to 20 mass%.

In a case of using the group A and the group B in combination, when the group A is a compound having a low threshold, group A: group B is preferably 0.0005:99.9995 to 50:50, more preferably 1:99 to 40:60, and still more preferably 10:90 to 30:70. When the group A is a compound not having a low threshold, group A: group B is preferably 1:99 to 99:1, more preferably 10:90 to 90:10, and still more preferably 30:70 to 70:30.

A fragrance composition containing the above group A and group B reduces discomfort due to at least one of body odor, scalp odor, bathroom odor, and nursing care odor all of which contains hexanal odor.

The fragrance composition according to the present invention can contain known fragrances or commonly used additives as described below within a range where the effects of the present invention are not impaired, that is, within a quantitative and qualitative range where the discomfort due to at least one of malodor selected from body odor, scalp odor, bathroom odor, and nursing care odor can be eliminated.

Examples of the known fragrances include: hydrocarbons such as α-pinene and cedrene; aliphatic saturated or unsaturated alcohols such as 1-octen-3-ol and nonanol; saturated or unsaturated chain terpene alcohols such as myrcenol and tetrahydrolinalool; cyclic terpene alcohols such as α-terpineol and bornylmethoxycyclohexanol; sesquiterpene alcohols such as farnesol; aromatic alcohols such as γ-phenylpropyl alcohol and dimethylbenzyl carbinol; aliphatic aldehydes; terpene-based aldehydes; aromatic aldehydes; aliphatic ketones; terpene cyclic ketones; cyclic ketones; aliphatic esters; acetate esters; propionate esters; butyrate esters; benzoate esters; phenylacetate esters; salicylate esters; and anthranilate esters. Blending amounts of these known fragrances in the present fragrance composition can be appropriately selected depending on the type of the fragrance to be blended, the purpose, or the like, and is not particularly limited.

The fragrance composition according to the present invention can be blended into perfume cosmetics and the like together with commonly used additives depending on the form and dosage form.

Examples of the commonly used additives include: known ultraviolet absorbers; cooling agents such as menthol and methyl salicylate; and skin irritants such as capsicum tincture and nonylvanillylamide. Blending amounts of these commonly used additives are not particularly limited, and are generally in a range of 0.005 mass% to 5.0 mass% based on the entire fragrance composition according to the present invention.

In addition, examples of the product containing the fragrance composition according to the present invention include a fragrance product, a perfume cosmetic, a basic cosmetic, a finishing cosmetic, a hair cosmetic, a suntan cosmetic, a medicated cosmetic, a hair care product, a soap, a body wash, a bath agent, a laundry detergent, a fabric softener, a cleaner, a kitchen detergent, a bleach, an aerosol agent, a deodorant/fragrance, a repellent, and a miscellaneous good. Examples of the fragrance product include perfumes, eau de parfum, eau de toilette, and cologne. Examples of the basic cosmetic include face wash cream, vanishing cream, cleansing cream, cold cream, massage cream, emulsion, lotion, serum, pack, and a makeup remover. Examples of the finishing cosmetic include foundation, lipstick, and lip balm. Examples of the hair cosmetic include a hair tonic, a hair liquid, and a hair spray. Examples of the suntan cosmetic include a suntan product and a sunscreen product. Examples of the medicated cosmetic include an antiperspirant, an aftershave lotion and gel, a permanent waving agent, a medicated soap, a medicated shampoo, and a medicated skin cosmetic. Examples of the hair care product include a shampoo, a rinse, a rinse-in-shampoo, a conditioner, a treatment, and a hair pack. Examples of the soap include a toilet soap and a bath soap. Examples of the body wash include a body soap, a body shampoo, and a hand soap. Examples of the bath agent include a bath additive (bath salts, bath tablets, bath liquids, etc.), a foam bath (bubble baths, etc.), bath oil (bath perfumes, bath capsules, etc.), a milk bath, a bath jelly, and a bath cube.

Examples of the detergent include a heavy laundry detergent, a light laundry detergent, a liquid detergent, a laundry soap, a compact detergent, and a powder soap. Examples of the fabric softener include a softener and a furniture care. Examples of the cleaner include a cleanser, a house cleaner, a toilet cleaner, a bathroom cleaner, a glass cleaner, a mold remover, and a drain cleaner. Examples of the kitchen detergent include a kitchen soap, a synthetic kitchen soap, and a dish detergent. Examples of the bleach include an oxidizing bleach (a chlorine bleach, an oxygen bleach, etc.), a reducing bleach (a sulfur-based bleach, etc.), and an optical bleach. Examples of the aerosol agent include a spray type and a powder spray. Examples of the deodorant/fragrance include a solid type, a gel type, and a liquid type. Examples of the miscellaneous good include various forms such as tissue paper and toilet paper.

In the case of being used in the above product, the fragrance composition according to the present invention is used as it is, or used in any desired state selected depending on the purpose, such as: a liquid state dissolved in polyhydric alcohols such as alcohol, propylene glycol, and glycerin; a solubilized state or a dispersed state solubilized or dispersed using surfactants such as a nonionic surfactant, an anionic surfactant, a cationic surfactant, and an amphoteric surfactant; or a microcapsule state obtained by a treatment with an encapsulating agent.

Further, the fragrance composition may be stabilized and released in a sustained manner by being included in an inclusion agent such as cyclodextrin. These are appropriately selected and used depending on the form of the final product, for example, a liquid form, a solid form, a powder form, a gel form, a mist form, or an aerosol form.

An amount of the fragrance composition in the product is in a range of 0.01 mass% to 100 mass%, preferably 0.01 mass% to 50.0 mass%, and more preferably 0.01 mass% to 20.0 mass%.

Further, it is not an odorant per se, but is used as a pro-fragrance with the ability to release the above fragrance composition under conditions of use/application. In this case, the blending amount of the fragrance composition is not particularly limited, and is generally in a range of 0.001 mass% to 20.0 mass % based on the entire product.

### Examples

Hereinafter, the present invention will be described in more detail with reference to Examples, but the present invention is not limited to these Examples in any way.

### Example 1

### <Evaluation of Malodor Prevention Ability of Group A>

The malodor prevention ability of a test substance was confirmed by sensory evaluation. As malodor, 10 µL of hexanal diluted 100 times with dipropylene glycol and 1 µL of the test substance were dropped into a cotton ball, which was charged into a plastic bottle. The bottle was left standing at room temperature for 1 hour to allow odor molecules to sufficiently volatilize inside the bottle. A sensory evaluation test was performed by a panel of 20 people, a malodor intensity when the malodor was dropped alone was rated as 8, and the odor intensity when the test substance was mixed was evaluated from 0 (no malodor was felt) to 10 (very strong malodor was felt). An average value was calculated from the obtained numerical values. The results are shown in Table 1.

When compounds in the group A and hexanal were sniffed simultaneously, all fragrance compounds had a significantly reduced hexanal odor intensity.

**Table 1**

| Fragrance compound | Hexanal odor intensity |
|---|---|
| Only hexanal | 8.0 |
| COOLACT 10 | 3.3 |
| Guava coeur, 0.1% triethyl citrate solution | 3.1 |
| Pamplefix, 50% triethyl citrate solution | 4.1 |
| Ambretone | 3.5 |
| Thesaron | 2.6 |
| Isopulegol | 3.4 |
| Hindinol | 3.9 |
| Dextramber | 3.0 |
| Orbitone | 3.9 |
| Phellandrene | 4.1 |
| Hedione | 4.2 |
| α-isomethylionone | 3.1 |
| Cedar leaf oil | 3.5 |
| Bergamot oil | 1.9 |
| Cardamom oil | 2.4 |
| Davana oil | 3.3 |
| Gaiac wood oil | 3.8 |
| Vetiver oil | 2.9 |
| Menthone | 6.9 |
| Carvone | 7.1 |

### Example 2

### <Evaluation of Malodor Prevention Ability by Combination of Group A and Group B>

Fragrance compositions of fragrances 1 to 60 were prepared according to formulations shown in Tables 2 to 8 below. TEC in the tables indicates triethyl citrate. The group B was divided into five groups as shown in Table 9, designated as group B1 to group B5, and added to the fragrance composition. Note that, a grouping method and the ratio within the groups are optional and are not limited by Table 9. Floral bases in Table 2 to Table 8 were prepared according to Table 10.

The malodor prevention ability of these fragrance compositions was confirmed by sensory evaluation. As for malodor, model body odor and model nursing care odor containing hexanal as one of key components were prepared according to Table 11 and Table 12. 10 µL of each model malodor and 1 µL of the fragrances 1 to 60 as test substances were dropped into a cotton ball, which was charged into a plastic bottle. The bottle was left standing at room temperature for 30 minutes to allow odor molecules to sufficiently volatilize inside the bottle. A sensory evaluation test was performed by a panel of 20 people, the discomfort when the malodor was dropped alone was rated as -3, and the discomfort when the test substance was mixed was evaluated from -4 (extremely discomfortable) to +4 (extremely comfortable). An average value was calculated from the obtained numerical values. The results are shown in Table 2 to Table 8.

A fragrance composition containing COOLACT 10 reduced discomfort due to the model body odor. Further, it was clear that in the case of use in combination with the group B1, a better effect of preventing the model body odor was exhibited. This prevention of model body odor was more remarkable than a case where a control substance (menthone, carvone) was mixed. Note that when the effect of other substances (Guava coeur and a 0.1% TEC solution, Pamplefix and a 50% TEC solution, Ambretone, Thesaron, isopulegol, Hindinol, Dextramber, Orbitone, Phellandrene, Hedione, α-isomethylionone, cedar leaf oil, bergamot oil, cardamom oil, davana oil, gaiac wood oil, and vetiver oil) on reducing the discomfort due to malodor was versified, it was clear that all substances reduced the discomfort due to each malodor, and in the case of use in combination with the group B1, an even better prevention effect was exhibited.

Similar verification was performed by replacing the group B1 in this verification with the group B2. As a result, it was clear that, similar to the case where the group B1 was used, in the case of use in combination with the group B2, a better effect of reducing the discomfort due to the model body odor was exhibited.

When similar verification was performed out by replacing the group B1 with the groups B3, B4, and B5, it was clear that in the case of use in combination with the group B, a better effect of reducing the discomfort due to the model body odor was exhibited, similar to the case of using the group B1.

Similar verification was performed by replacing, as the malodor, the model body odor with the model nursing care odor. As a result, it was clear that the group A reduced the discomfort due to the model nursing care odor, and in the case of use in combination with the groups B1 to B5, an even better effect of reducing the model nursing care odor was exhibited.

**Table 2**

| Fragrance component (unit: mass%) | | Test number | | | | | | | | |
|---|---|---|---|---|---|---|---|---|---|---|
| | | 1 | 2 | 3 | 4 | 5 | 6 | 7 | 8 | 9 |
| COOLACT 10 | | 1.0 | 1.0 | 1.0 | | | | | | |
| Guava coeur, 0.1% triethyl citrate solution | | | | | 0.1 | 0.1 | 0.1 | | | |
| Pamplefix, 50% triethyl citrate solution | | | | | | | | 0.5 | 0.5 | 0.5 |
| Group B1 | | | 1.5 | | | 1.5 | | | 1.5 | |
| Group B2 | | | | 1.5 | | | 1.5 | | | 1.5 |
| Floral base | | Balance | | | | | | | | |
| Total | | 100 | 100 | 100 | 100 | 100 | 100 | 100 | 100 | 100 |
| Comfort/discomfort level | Body odor | 2.4 | 3.1 | 2.9 | 2.5 | 2.9 | 2.9 | 1.7 | 2.7 | 2.5 |
| | Nursing care odor | 2.5 | 2.9 | 3.0 | 2.6 | 3.1 | 2.9 | 1.8 | 2.1 | 2.4 |

**Table 3**

| Fragrance component (unit: mass%) | | Test number | | | | | | | | |
|---|---|---|---|---|---|---|---|---|---|---|
| | | 10 | 11 | 12 | 13 | 14 | 15 | 16 | 17 | 18 |
| Ambretone | | 1.0 | 1.0 | 1.0 | | | | | | |
| Thesaron | | | | | 1.0 | 1.0 | 1.0 | | | |
| Isopulegol | | | | | | | | 1.0 | 1.0 | 1.0 |
| Group B1 | | | 1.5 | | | 1.5 | | | 1.5 | |
| Group B2 | | | | 1.5 | | | 1.5 | | | 1.5 |
| Floral base | | Balance | | | | | | | | |
| Total | | 100 | 100 | 100 | 100 | 100 | 100 | 100 | 100 | 100 |
| Comfort/discomfort level | Body odor | 2.2 | 2.8 | 2.7 | 2.9 | 3.6 | 3.5 | 2.3 | 3.0 | 2.7 |
| | Nursing care odor | 2.0 | 2.7 | 2.5 | 2.7 | 3.3 | 3.3 | 2.1 | 2.7 | 2.9 |

**Table 4**

| Fragrance component (unit: mass%) | | Test number | | | | | | | | |
|---|---|---|---|---|---|---|---|---|---|---|
| | | 19 | 20 | 21 | 22 | 23 | 24 | 25 | 26 | 27 |
| Hindinol | | 0.5 | 0.5 | 0.5 | | | | | | |
| Dextramber | | | | | 0.1 | 0.1 | 0.1 | | | |
| Orbitone | | | | | | | | 1.0 | 1.0 | 1.0 |
| Group B1 | | | 1.5 | | | 1.5 | | | 1.5 | |
| Group B2 | | | | 1.5 | | | 1.5 | | | 1.5 |
| Floral base | | Balance | | | | | | | | |
| Total | | 100 | 100 | 100 | 100 | 100 | 100 | 100 | 100 | 100 |
| Comfort/discomfort level | Body odor | 1.9 | 2.5 | 2.5 | 2.6 | 2.9 | 3.0 | 1.9 | 2.5 | 2.8 |
| | Nursing care odor | 1.8 | 2.4 | 2.5 | 2.5 | 3.1 | 3.1 | 2.0 | 2.8 | 2.6 |

**Table 5**

| Fragrance component (unit: mass%) | | Test number | | | | | | | | |
|---|---|---|---|---|---|---|---|---|---|---|
| | | 28 | 29 | 30 | 31 | 32 | 33 | 34 | 35 | 36 |
| Phellandrene | | 0.5 | 0.5 | 0.5 | | | | | | |
| Hedione | | | | | 1.0 | 1.0 | 1.0 | | | |
| α-isomethylionone | | | | | | | | 1.0 | 1.0 | 1.0 |
| Group B1 | | | 1.5 | | | 1.5 | | | 1.5 | |
| Group B2 | | | | 1.5 | | | 1.5 | | | 1.5 |
| Floral base | | Balance | | | | | | | | |
| Total | | 100 | 100 | 100 | 100 | 100 | 100 | 100 | 100 | 100 |
| Comfort/discomfort level | Body odor | 1.7 | 2.4 | 2.6 | 1.6 | 2.5 | 2.3 | 2.5 | 2.9 | 2.8 |
| | Nursing care odor | 1.8 | 2.5 | 2.2 | 2.0 | 2.5 | 2.3 | 2.3 | 2.6 | 2.7 |

**Table 6**

| Fragrance component (unit: mass%) | | Test number | | | | | | | | |
|---|---|---|---|---|---|---|---|---|---|---|
| | | 37 | 38 | 39 | 40 | 41 | 42 | 43 | 44 | 45 |
| Cedar leaf oil | | 1.0 | 1.0 | 1.0 | | | | | | |
| Bergamot oil | | | | | 1.0 | 1.0 | 1.0 | | | |
| Cardamom oil | | | | | | | | 1.0 | 1.0 | 1.0 |
| Group B1 | | | 1.5 | | | 1.5 | | | 1.5 | |
| Group B2 | | | | 1.5 | | | 1.5 | | | 1.5 |
| Floral base | | Balance | | | | | | | | |
| Total | | 100 | 100 | 100 | 100 | 100 | 100 | 100 | 100 | 100 |
| Comfort/discomfort level | Body odor | 2.2 | 2.6 | 2.7 | 3.5 | 4.1 | 4.0 | 3.1 | 3.4 | 3.2 |
| | Nursing care odor | 2.0 | 2.5 | 2.8 | 3.3 | 3.9 | 3.8 | 3.2 | 3.7 | 4.0 |

**Table 7**

| Fragrance component (unit: mass%) | | Test number | | | | | | | | |
|---|---|---|---|---|---|---|---|---|---|---|
| | | 46 | 47 | 48 | 49 | 50 | 51 | 52 | 53 | 54 |
| Davana oil | | 1.0 | 1.0 | 1.0 | | | | | | |
| Gaiac wood oil | | | | | 0.5 | 0.5 | 0.5 | | | |
| Vetiver oil | | | | | | | | 1.0 | 1.0 | 1.0 |
| Group B1 | | | 1.5 | | | 1.5 | | | 1.5 | |
| Group B2 | | | | 1.5 | | | 1.5 | | | 1.5 |
| Floral base | | Balance | | | | | | | | |
| Total | | 100 | 100 | 100 | 100 | 100 | 100 | 100 | 100 | 100 |
| Comfort/discomfort level | Body odor | 2.4 | 3.0 | 2.9 | 2.0 | 2.6 | 2.2 | 2.7 | 3.0 | 3.0 |
| | Nursing care odor | 2.5 | 2.9 | 3.1 | 2.1 | 2.5 | 2.5 | 2.5 | 2.9 | 3.2 |

**Table 8**

| Fragrance component (unit: mass%) | | Test number | | | | | |
|---|---|---|---|---|---|---|---|
| | | 55 | 56 | 57 | 58 | 59 | 60 |
| Menthone | | 1.0 | 1.0 | 1.0 | | | |
| Carvone | | | | | 1.0 | 1.0 | 1.0 |
| Group B1 | | | 1.5 | | | 1.5 | |
| Group B2 | | | | 1.5 | | | 1.5 |
| Floral base | | Balance | | | | | |
| Total | | 100 | 100 | 100 | 100 | 100 | 100 |
| Comfort/discomfort level | Body odor | -1.5 | -1.3 | -1.6 | -1.8 | -1.7 | -2.0 |
| | Nursing care odor | -1.2 | -1.5 | -1.1 | -1.5 | -1.3 | -1.6 |

**Table 9**

| Group | Group B: fragrance component |
|---|---|
| Group B1 | Limonene, linalool, linalyl acetate, geraniol, citronellal, citronella java oil, and grapefruit oil |
| Group B2 | Benzyl acetate, 3-methyl-2-(cis-2-pentenyl)-2-cyclopenten-1-one, and citronellol |
| Group B3 | β-damascone, γ-decalactone, prenyl acetate, and isoamyl acetate |
| Group B4 | Allyl hexanoate, ethyl hexanoate, ethyl isovalerate, cis-2-methyl-4-propyl-1,3-oxathiane, and ethyl amyl ketone |
| Group B5 | Para-menth-1,8-dien-7-al, (3-methylbutyloxy)-2-propenyl acetate, cis-3-hexenyl acetate, 2-phenylpropanal, nutmeg oil, coriander oil, and galbanum oil |

**Table 10**

| Fragrance component | Unit: mass% |
|---|---|
| Allyl heptanoate | 4 |
| 2-Ethyl-4-(2,2,3-trimethyl-3-cyclopenten-1-yl)-2-buten-1-ol | 2 |
| Benzyl salicylate | 1 |
| Tricyclodecenyl propionate | 4 |
| Dihydromyrcenol | 11 |
| Dimethylbenzylcarbinyl acetate | 4 |
| 2-Isobutyl-4-methyloxan-4-ol | 13 |
| Galaxolide | 20 |
| Geranyl acetate | 3 |
| Hexyl salicylate | 1 |
| Hexyl acetate | 7 |
| Hexyl cinnamaldehyde | 6 |
| Manzanate | 2 |
| 1-Phenylethyl acetate | 3 |
| γ-undecalactone | 2 |
| 2-(Tert-butyl)cyclohexyl acetate | 7 |
| Rose base (manufactured by Takasago International Corporation) | 10 |
| Total | 100 |

**Table 11**

| Component | Unit mass% |
|---|---|
| 3-Hydroxy-3-methylhexanoic acid | 10.0 |
| 3-Methyl-2-hexenoic acid | 5.0 |
| Acetic acid | 11.7 |
| Isobutanoic acid | 4.0 |
| Butanoic acid | 0.8 |
| Isovaleric acid | 4.0 |
| Hexanoic acid | 0.8 |
| Heptanoic acid | 0.8 |
| Octanoic acid | 0.8 |
| Nonanoic acid | 0.8 |
| Lactic acid | 55.5 |
| Hexanal | 4.0 |
| Octanal | 0.4 |
| Nonanal | 0.1 |
| Decanal | 0.5 |
| Undecanal | 0.6 |
| Dodecanal | 0.2 |
| Total | 100.0 |

**Table 12**

| Component | Unit: mass% |
|---|---|
| p-Cresol | 0.1 |
| Decanal | 2.0 |
| Nonanal | 2.0 |
| Octanal | 2.0 |
| Heptanal | 2.0 |
| Hexanal | 2.0 |
| Ethanol | 89.9 |
| Total | 100.0 |

### Example 3

### <Gel Aromatic Agent>

A floral fragrance composition (Test number 2 in Table 2) containing 1.0 mass% of COOLACT 10 and 1.5 mass% of the group B1 was used as a fragrance composition to produce a gel aromatic agent by a known method according to the formulation shown in Table 13 below. Note that a gel aromatic agent was similarly produced by a known method using a fragrance composition having a formulation in which the COOLACT 10 was replaced with any compound in the group A, and the group B was replaced with any compound in the group B.

**Table 13**

| Component | Unit: mass% |
|---|---|
| Carrageenan | 2.0 |
| Locust bean gum | 0.2 |
| Propylene glycol | 2.0 |
| POE (20) sorbitan monostearate | 0.5 |
| Fragrance composition | 5.0 |
| Purified water | Remainder |
| Total | 100.0 |

### Example 4

### <Body Soap>

A floral fragrance composition containing 1.0 mass% of Ambretone, 1.5 mass% of the group B2, and a floral base as the remainder was used as a fragrance composition to produce a body soap by a known method. The formulation of a body soap base is shown in Table 14. Note that a body soap was similarly produced by a known method using a fragrance composition having a formulation in which the Ambretone was replaced with any compound in the group A, and the group B was replaced with any compound in the group B.

**Table 14**

| Component | Unit: mass% |
|---|---|
| Triethanolamine | 9.0 |
| Lauric acid | 6.0 |
| Myristic acid | 9.0 |
| Disodium lauryl polyoxyethylene sulfosuccinate (1E.O.) (42%) | 10.0 |
| Alkyl (C8-16) glucoside | 8.0 |
| Glyceryl laurate | 1.0 |
| 2-Hydroxyethyl distearate | 2.5 |
| Coconut oil fatty acid diethanolamide | 3.0 |
| Propylene glycol | 5.0 |
| Dibutylhydroxytoluene | 0.1 |
| Edetate disodium | 0.1 |
| Ethyl paraoxybenzoate | 0.2 |
| Methyl paraoxybenzoate | 0.1 |
| Fragrance composition | 1.0 |
| Purified water | Remainder |
| Total | 100.0 |

### Example 5

### <Shampoo>

A floral fragrance composition containing 1.0 mass% of Thesaron, 1.5 mass% of the group B3, and a floral base as the remainder was used as a fragrance composition to produce a shampoo by a known method. The formulation of a shampoo base is shown in Table 15. Note that a shampoo was similarly produced by a known method using a fragrance composition having a formulation in which the Thesaron was replaced with any compound in the group A, and the group B was replaced with any compound in the group B.

**Table 15**

| Component | Unit: mass% |
|---|---|
| Sodium polyoxyethylene lauryl ether sulfate | 28.0 |
| Cocamidopropyl betaine | 8.0 |
| Methylparaben | 2.0 |
| Phenoxyethanol | 0.3 |
| Sodium lauryl sulfate | 28.0 |
| Fragrance composition | 1.0 |
| Purified water | Remainder |
| Total | 100.0 |

### Example 6

### <Conditioner>

A floral fragrance composition containing 1.0 mass% of isopulegol, 1.5 mass% of the group B4, and a floral base as the remainder was used as a fragrance composition to produce a conditioner by a known method. The formulation of a conditioner base is shown in Table 16. Note that a conditioner was similarly produced by a known method using a fragrance composition having a formulation in which the isopulegol was replaced with any compound in the group A, and the group B was replaced with any compound in the group B.

**Table 16**

| Component | Unit: mass% |
|---|---|
| Behentrimonium chloride | 1.7 |
| Cetanol | 1.0 |
| Polysorbate 80 | 1.0 |
| Stearyl alcohol | 3.0 |
| Phenoxyethanol | 1.0 |
| BELSIL CDM 3526 VP | 2.0 |
| EDTA-4Na | 0.4 |
| Hydroxyethylcellulose | 0.2 |
| Stearamidopropyl dimethylamine | 1.0 |
| Citric acid | 2.0 |
| Fragrance composition | 1.0 |
| Purified water | Remainder |
| Total | 100.0 |

### Example 7

### <Liquid Detergent>

A floral fragrance composition containing 0.5 mass% of Hindinol, 1.5 mass% of the group B5, and a floral base as the remainder was used as a fragrance composition to produce a liquid detergent by a known method. The formulation of a liquid detergent base is shown in Table 17. Note that a liquid detergent was similarly produced by a known method using a fragrance composition having a formulation in which the Hindinol was replaced with any compound in the group A, and the group B was replaced with any compound in the group B.

**Table 17**

| Component | Unit: mass% |
|---|---|
| Polyoxyalkylene alkyl ether | 50.0 |
| Butyl carbitol | 8.0 |
| Linear alkylbenzene sulfonate | 2.5 |
| p-Toluenesulfonic acid | 1.0 |
| Polyethylene glycol | 1.0 |
| Monoethanolamine | 1.0 |
| Coconut oil fatty acid | 0.1 |
| Citric acid | 0.2 |
| Sodium benzoate | 0.5 |
| BHT | 0.1 |
| Fragrance composition | 1.0 |
| Water | Remainder |
| Total | 100.0 |

### Example 8

### <Liquid softener>

A floral fragrance composition containing 1.0 mass% of bergamot oil, 1.5 mass% of the group B1, and a floral base as the remainder was used as a fragrance composition to produce a liquid softener by a known method. The formulation of a liquid softener base is shown in Table 18. Note that a liquid softener was similarly produced by a known method using a fragrance composition having a formulation in which the bergamot oil was replaced with any compound in the group A, and the group B was replaced with any compound in the group B.

**Table 18**

| Component | Unit: mass% |
|---|---|
| Imidazoline type cationic surfactant | 6.00 |
| Ethylene glycol | 2.00 |
| Polyoxyethylene oleyl cetyl ether | 0.20 |
| Fragrance composition | 1.00 |
| Purified water | Remainder |
| Total | 100.00 |

Fragrance components in the Tables are shown as follows.
COOLACT 10: 3-(menthoxy)propane-1,2-diol
Guava coeur: ethyl 3-mercapto-2-methylbutyrate,
Pamplefix: dimethyl-2-(1-phenylethyl)cyclopropylmethanol,
Ambretone: 5-cyclohexadecenone,
Thesaron: ethyl 2,2,6-trimethylcyclohexane-1-carboxylate,
Hindinol: 2-methyl-4-(2,2,3-trimethyl-3-cyclopentenyl)-2-buten-1-ol,
Dextramber: 1-(2,2,6-trimethylcyclohexyl)hexan-3-ol,
Orbitone: 2-acetyl-2,3,8,8-tetramethyloctarine,
Phellandrene: p-mentha-1,5-diene,
Hedione: methyl (2-pentyl-3-oxo-cyclopentyl) acetate,
α-isomethylionone: 3-methyl-4-(2,6,6-trimethyl-2-cyclohexen-1-yl)-3-buten-2-one

## Claims

1. A fragrance composition for reducing discomfort due to malodor, the fragrance composition comprising: at least one selected from the group consisting of fragrance components in the following group A.
Group A: 3-(menthoxy)propane-1,2-diol, ethyl 3-mercapto-2-methylbutyrate, dimethyl-2-(1-phenylethyl)cyclopropylmethanol, 5-cyclohexadecenone, ethyl 2,2,6-trimethylcyclohexane-1-carboxylate, isopulegol, 2-methyl-4-(2,2,3-trimethyl-3-cyclopentenyl)-2-buten-1-ol, 1-(2,2,6-trimethylcyclohexyl)hexan-3-ol, 2-acetyl-2,3,8,8-tetramethyloctarine, p-mentha-1,5-diene, methyl (2-pentyl-3-oxo-cyclopentyl) acetate, 3-methyl-4-(2,6,6-trimethyl-2-cyclohexen-1-yl)-3-buten-2-one, cedar leaf oil, bergamot oil, cardamom oil, davana oil, gaiac wood oil, and vetiver oil

2. The fragrance composition according to claim 1, wherein the malodor is at least one selected from the group consisting of body odor, scalp odor, bathroom odor, and nursing care odor.

3. The fragrance composition according to claim 1, wherein the malodor is hexanal odor.

4. The fragrance composition according to any one of claims 1 to 3, further comprising: at least one selected from the group consisting of fragrance components in the following group B.
Group B: limonene, linalool, linalyl acetate, geraniol, citronellal, β-damascone, γ-decalactone, prenyl acetate, isoamyl acetate, benzyl acetate, 3-methyl-2-(cis-2-pentenyl)-2-cyclopenten-1-one, citronellol, allyl hexanoate, ethyl hexanoate, ethyl isovalerate, cis-2-methyl-4-propyl-1,3-oxathiane, ethyl amyl ketone, para-menth-1,8-dien-7-al, (3-methylbutyloxy)-2-propenyl acetate, cis-3-hexenyl acetate, 2-phenylpropanal, citronella java oil, grapefruit oil, nutmeg oil, coriander oil, and galbanum oil

5. The fragrance composition according to claim 4, wherein the fragrance component in the group A is contained in an amount of 0.000001 mass% to 50 mass%, and the fragrance component in the group B is contained in an amount of 0.0001 mass% to 50 mass%.

6. A product comprising: the fragrance composition according to any one of claims 1 to 5 in an amount of 0.01 mass% to 100 mass%.

7. A method for reducing discomfort due to at least one of malodor selected from the group consisting of body odor, scalp odor, bathroom odor, and nursing care odor, the method comprising: applying the fragrance composition according to any one of claims 1 to 5 to at least one of malodor selected from the group consisting of body odor, scalp odor, bathroom odor, and nursing care odor to reduce discomfort due to malodor.

8. A use of a fragrance composition for reducing discomfort due to malodor, the fragrance composition comprising: at least one selected from the group consisting of fragrance components in the following group A.
Group A: 3-(menthoxy)propane-1,2-diol, ethyl 3-mercapto-2-methylbutyrate, dimethyl-2-(1-phenylethyl)cyclopropylmethanol, 5-cyclohexadecenone, ethyl 2,2,6-trimethylcyclohexane-1-carboxylate, isopulegol, 2-methyl-4-(2,2,3-trimethyl-3-cyclopentenyl)-2-buten-1-ol, 1-(2,2,6-trimethylcyclohexyl)hexan-3-ol, 2-acetyl-2,3,8,8-tetramethyloctarine, p-mentha-1,5-diene, methyl (2-pentyl-3-oxo-cyclopentyl) acetate, 3-methyl-4-(2,6,6-trimethyl-2-cyclohexen-1-yl)-3-buten-2-one, cedar leaf oil, bergamot oil, cardamom oil, davana oil, gaiac wood oil, and vetiver oil

9. The use of the fragrance composition for reducing discomfort due to malodor according to claim 8, wherein the malodor is at least one selected from the group consisting of body odor, scalp odor, bathroom odor, and nursing care odor.

10. The use of the fragrance composition for reducing discomfort due to malodor according to claim 8, wherein the malodor is hexanal odor.

11. The use of the fragrance composition for reducing discomfort due to malodor according to any one of claims 8 to 10, the fragrance composition further comprising: at least one selected from the group consisting of fragrance components in the following group B.
Group B: limonene, linalool, linalyl acetate, geraniol, citronellal, β-damascone, γ-decalactone, prenyl acetate, isoamyl acetate, benzyl acetate, 3-methyl-2-(cis-2-pentenyl)-2-cyclopenten-1-one, citronellol, allyl hexanoate, ethyl hexanoate, ethyl isovalerate, cis-2-methyl-4-propyl-1,3-oxathiane, ethyl amyl ketone, para-menth-1,8-dien-7-al, (3-methylbutyloxy)-2-propenyl acetate, cis-3-hexenyl acetate, 2-phenylpropanal, citronella java oil, grapefruit oil, nutmeg oil, coriander oil, and galbanum oil
